Europäisches Patentamt

European Patent Office

Office européen des brevets

(11) Veröffentlichungsnummer: **0 233 986 B1**

# EUROPÄISCHE PATENTSCHRIFT

(45) Veröffentlichungstag der Patentschrift: **25.09.91**

(51) Int. Cl.⁵: **A61M 25/00**

(21) Anmeldenummer: **86110936.1**

(22) Anmeldetag: **07.08.86**

(54) **Intraperitonealkatheter für die Zuführung flüssiger Arzneimittel, insbesondere von Insulin.**

(30) Priorität: **12.08.85 DE 3528878**

(43) Veröffentlichungstag der Anmeldung:
**02.09.87 Patentblatt 87/36**

(45) Bekanntmachung des Hinweises auf die
Patenterteilung:
**25.09.91 Patentblatt 91/39**

(84) Benannte Vertragsstaaten:
**DE FR GB IT**

(56) Entgegenhaltungen:
**EP-A- 0 047 013**
**EP-A- 0 119 596**
**US-A- 4 256 102**

(73) Patentinhaber: **Fresenius AG**
**Borkenberg 14**
**W-6370 Oberursel/Taunus 1(DE)**

(72) Erfinder: **von Sanden, Hasko, Dr. med.**
**Dipl.-Ing.**
**Kirchenstrasse 25**
**W-8000 München 80(DE)**

(74) Vertreter: **von Puttkamer, Nikolaus, Dipl.-Ing.**
**Pienzenauerstrasse 2**
**W-8000 München 80(DE)**

## Beschreibung

Die Erfindung betrifft einen Intraperitonealkatheter für die Zuführung flüssiger Arzneimittel, insbesondere von Insulin, nach dem Oberbegriff des Patentanspruches 1.

Aus der DE-PS 31 02 993 ist eine subkutan implantierbare Vorrichtung für die Zuführung flüssiger Arzneimittel bekannt, die im wesentlichen aus einer Kammer mit einer Öffnung besteht, die durch eine flache Membran aus einem elastischen Material verschlossen ist. Mit der Kammer ist ein Schlauch verbunden, dessen Ende im subkutan implantierten Zustand in die Bauchhöhle eines Patienten hineinragt. Beispielsweise wird mit einer derartigen Vorrichtung Insulin subkutan in die Bauchhöhle geleitet. Hierzu wird das Insulin über die praktisch parallel zur Haut verlaufende Durchstechmembran in die Kammer eingebracht. Ein Problem besteht bei einer derartigen Vorrichtung darin, daß unkontrollierbare Insulinabgaben in gefährlichen Dosen möglich sind, wenn das in der Kammer befindliche Insulin sich beispielsweise bei einer Temperaturerhöhung ausdehnt. Eine unkontrollierte Insulinabgabe ist auch beispielsweise dann möglich, wenn der Körper des Insulinkranken, in den die bekannte Vorrichtung implantiert wurde, einer Erschütterung ausgesetzt wird.

Aus der EP-A-119 596 geht ein implantierbarer Zuspritzkatheter hervor, bei dem die zuvor genannten Probleme ebenfalls auftreten können, weil der Katheterkopf so ausgebildet ist, daß zwischen der Mittelöffnung des mit dem Katheterkopf verbundenen Katheters und der Einstechmembran eine Kammer besteht, die zu den beschriebenen unkontrollierten Insulinabgaben in gefährlichen Dosen führen kann.

Die Aufgabe der vorliegenden Erfindung besteht daher darin, einen Intraperitonealkatheter der eingangs genannten Art dahingehend zu verbessern, daß unerwünschte bzw. schädliche Abgaben bzw. Zuführungen flüssiger Arzneimittel sicher vermeidbar sind.

Diese Aufgabe wird durch einen wie eingangs genannten Intraperitonealkatheter gelöst, der durch die in dem kennzeichnenden Teil des Patentanspruches 1 angegebenen Merkmale gekennzeichnet ist.

Ein wesentlicher Vorteil der vorliegenden Erfindung besteht darin, daß durch sie bei der Verabreichung von flüssigen Arzneimitteln, insbesondere von Insulin, für die Gesundheit des Patienten gefährliche Abgabemengen vermieden werden können.

Vorteilhafterweise kann bei der Verwendung des erfindungsgemäßen Intraperitonealkatheters eine externe Arzneimittelpumpe direkt in einer äußerst einfachen Weise mit dem unter der Haut eines Patienten befindlichen Katheterkopf derart sicher verbunden werden, daß die Arzneimittelzufuhr direkt zur Längsöffnung des Katheterrohres von einer in den Katheterkopf eingeführten Einstechnadel erfolgt, ohne daß schädliche Toträume bestehen.

Ein weiterer Vorteil besteht darin, daß der erfindungsgemäße Katheter einfach aufgebaut, billig und leicht herstellbar und einfach handhabbar ist.

Vorteilhafterweise ist der erfindungsgemäße Katheter so beschaffen, daß über ihn über einen längeren Zeitraum, beispielsweise ein halbes Jahr, täglich Arzneimittel, insbesondere Insulin, von außen zuführbar sind.

Im folgenden werden die Erfindung und deren Ausgestaltungen im Zusammenhang mit den Figuren naher erläutert. Es zeigt:

Fig. 1        einen erfindungsgemäßen Intraperitonealkatheter;

Fig. 2        die vergrößerte Darstellung des Kopfes des Katheters der Fig. 1; und

Fig. 3 und 4        eine Weiterbildung der Erfindung.

Zu der Erfindung führten die folgenden Überlegungen. Um die eingangs genannten Nachteile bekannter Intraperitonealkatheter zu vermeiden, soll der mit dem Katheterrohr verbundene Katheterkopf so beschaffen sein, daß er eine direkte Verbindung zwischen der Mittelöffnung des Katheterrohres bzw. Katheterschlauches und einer in den Katheterkopf eingeführten Einstechnadel ermöglicht, die mit einer externen Abgabeeinrichtung für flüssige Arzneimittel verbunden ist. Dabei soll zwischen der in den Katheterkopf eingeführten Spitze der Einstechnadel und der Mittelöffnung ein möglichst nahtloser Übergang bestehen, um jegliche Toträume zu vermeiden, die zu unerwünschten Arzneimittelansammlungen führen könnten. Derartige unerwünschte Arzneimittelvolumen könnten nämlich, wie dies eingangs bereits erwähnt wurde, beispielsweise bei Temperaturschwankungen und bei mechanischen Einwirkungen zu unerwünschten und schädlichen Arzneimittelabgabeschwankungen führen.

Allgemein ausgedrückt besteht der erfindungsgemäße Intraperitonealkatheter aus einem Katheterschlauch und einem vorzugsweise einstückig damit verbundenen Katheterkopf, der einen elastischen Einsatz besitzt, durch den eine Einstechnadel zu einer Mittelöffnung im Katheterkopf führbar ist, wobei die Mittelöffnung des Katheterkopfes in die Mittelöffnung des Katheterschlauches übergeht. Auf diese Weise wird sichergestellt, daß in dem Verbindungsweg zwischen der Mittelöffnung des Katheterrohres und der in den Katheterkopf eingeführten Einstechnadel, die direkt mit einer Abgabeeinrichtung für flüssige Arzneimittel, beispielsweise mit einer Insulinpumpe verbunden ist, keine Toträume bestehen, in denen sich Arzneimittelvolumen ansammeln könnten, die die obengenannten Nachteile bewirken könnten.

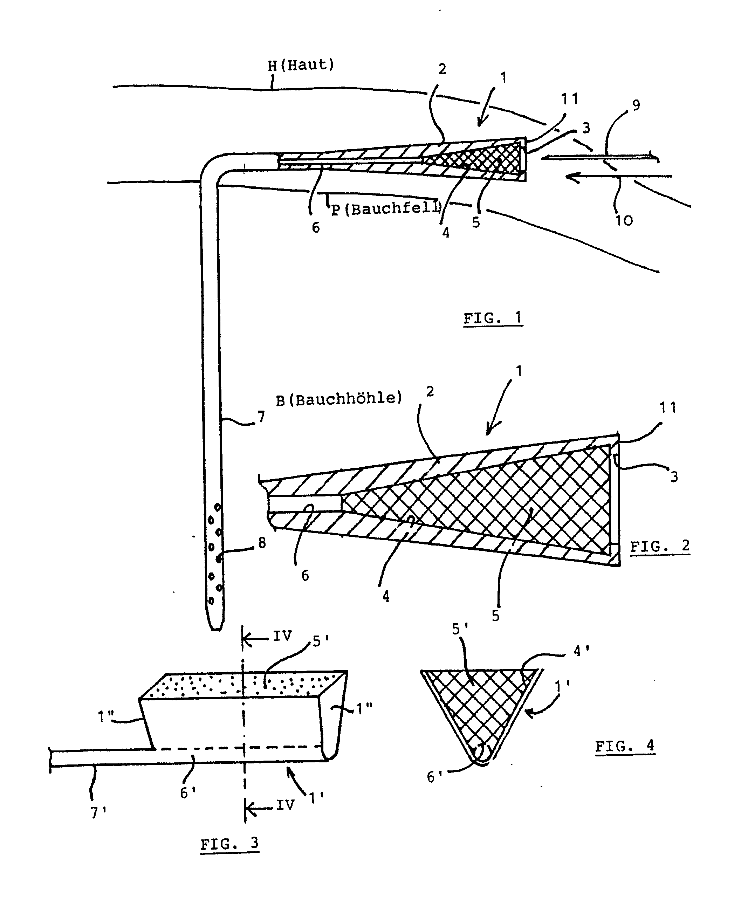

Die Fig. 1 und 2 zeigen eine erste bevorzugte Ausführungsform der Erfindung. Dabei ist ein vorzugsweise flexibler Katheterschlauch bzw. ein vorzugsweise flexibles Katheterrohr mit dem Bezugszeichen 7 bezeichnet. Dieses Rohr ragt nach der Implantation des vorliegenden Katheters in den Körper eines Patienten, vorzugsweise in die Bauchhöhle B zwischen die Därme. Dies ist insbesondere bei einer Insulinzufuhr mit dem vorliegenden Katheter von Vorteil, weil im Bereich der Bauchhöhle B eine rasche Resorption des Insulins erfolgt. Zum Anschluß des starren Katheterkopfes 1, der vorzugsweise einstückig mit dem Katheterrohr 7 verbunden ist, an eine externe Abgabeeinrichtung für flüssige Arzneimittel, insbesondere zum Anschluß an eine externe Insulinpumpe, weist der Katheterkopf 1 in seinem Inneren eine sich zu seinem freien Ende hin erweiternde konische bzw. trichterförmige Ausnehmung 4 auf, die sich zu dem freien Ende des Katheterkopfes 1 hin öffnet. In dieser Ausnehmung 4 befindet sich ein die Ausnehmung 4 ausfüllender Einsatz, der aus einem elastisch verformbaren Material, vorzugsweise aus Silikonkautschuk besteht. Der Katheterkopf 1 besteht beispielsweise aus einem Material, das ein Einwachsen des Kopfes in das Unterhautgewebe ermöglicht. Durch dieses Einwachsen kann verhindert werden, daß entlang dem einen Fremdkörper darstellenden, implantierten Katheter keine von der Einstichstelle in der Haut in den Körper bzw. in die freie Bauchhöhle wandern. Beispielsweise besteht der Katheterkopf aus Dacron®.

Um ein Herausgleiten des Einsatzes 5 aus der Ausnehmung 4 des Katheterkopfes 1 zum freien Ende des Kopfes 1 hin zu vermeiden, weist die Öffnung 3 des Katheterkopfes 1, durch die der Einsatz 5 in die Ausnehmung 4 eingebracht wird, vorzugsweise einen radial nach innen ragenden Flansch 11 auf, der eine Anschlagschulter für den in der Ausnehmung 4 befindlichen Einsatz 5 bildet. Es ist leicht möglich, beim Zusammenbau des Katheters den flexiblen Einsatz 5 durch die Öffnung 3 hindurchzuführen. Dabei wird der Einsatz 5 im Bereich der Öffnung 3 zusammengepreßt. Wenn sich der Einsatz 5 völlig in der Ausnehmung 4 befindet, hintergreift sein dem freien Ende des Kopfes 1 zugewandter Randbereich den Flansch 11. Es ist auch denkbar, den Einsatz 5 in der Ausnehmung 4 direkt zu verkleben. In diesem Fall muß der Katheterkopf 1 keinen radialen Flansch aufweisen.

Von wesentlicher Bedeutung ist es, daß, wie dies insbesondere aus der Fig. 2 hervorgeht, zwischen dem dem Rohr 7 zugewandten Ende des Einsatzes 5 bzw. der Ausnehmung 4 und der Mittelöffnung 6 kein Totraum besteht. Dies bedeutet, daß eine durch den Einsatz 5 eingesteckte Einstechnadel 9 (Pfeilrichtung 10), die mit der externen Arzneimittel- bzw. Insulinpumpe in Verbindung steht im wesentlichen direkt in die Mittelöffnung des Rohres 7 bzw. des Katheterkopfes 1 hineinragt bzw. mündet, so daß das aus der Einstechnadel 9 austretende Insulin, ohne vorher in einen Totraum zu gelangen, direkt in die Mittelöffnung 6 eintritt. Auf diese Weise wird sicher verhindert, daß sich in dem Körper ein Insulinvolumen befindet, daß bei einer plötzlichen Temperaturerhöhung und einer entsprechenden Ausdehnung, einer plötzlichen Temperaturerniedrigung und einer entsprechenden Verkleinerung oder bei einer plötzlichen Druckeinwirkung zu einer unerwünschten Insulinabgabe führt. Anders ausgedrückt ist mit dem vorliegenden Katheter ein direkter bzw. im wesentlicher nahtloser Übergang zwischen der Mittelöffnung 6 und der externen Insulinpumpe möglich, so daß lediglich die von der Insulinpumpe programmiert abgegebene Verabreichungseinheiten dem Körper gezielt zugeführt werden.

In an sich bekannter Weise kann das Rohr 7 an seinem im Bereich der Bauchhöhle B befindlichen Ende Öffnungen 8 zur Arzneimittelabgabe aufweisen.

Im folgenden wird die Handhabung des zuvor erläuterten Intraperitonealkatheters näher erläutert. Nach der Implantation des Katheters befindet sich, wie dies schon erlautert wurde, das Ende des Katheterrohres 7 in dem Bereich, in dem die Arzneimittelabgabe erfolgen soll, beispielsweise in der Bauchhöhle B. Der Katheterkopf 1 befindet sich unmittelbar unter der Haut des Patienten. Um eine externe Abgabeeinrichtung, beispielsweise eine Insulinpumpe, mit dem Katheterkopf 1 zu verbinden, wird dieser von außen mit einer Hand erfühlt und zusammen mit einer ihn umgebenden Hautfalte derart ergriffen, daß durch die Öffnung 3 und in den dahinter befindlichen Einsatz 5 eine mit einem nicht dargestellten Schlauch und über diesen mit der Insulinpumpe verbundene Einstechnadel 9 einführbar ist. Aufgrund der Konizität bzw. der Trichterform der Ausnehmung 4 wird die Spitze der Einstechnadel 9 zwangsweise zur Mittelöffnung 6 des Katheterkopfes 1 geführt, die in die Mittelöffnung 6 übergeht. Die Arzneimittelabgabe kann nun über den vorliegenden Katheter erfolgen. Zur Bewirkung einer Abkoppelung der Arzneimittelabgabeeinrichtung vom Katheter ist es lediglich erforderlich, die Einstechnadel 9 aus dem Einsatz 5 herauszuziehen, wobei die bestehende Öffnung nach dem Entfernen der Einstechnadel 9 aufgrund der Elastizität des Materials des Einsatzes 5 sofort verschlossen wird.

Im folgenden werden Bemessungsangaben für den zuvor erläuterten Katheter angegeben. Beispielsweise beträgt der Außendurchmesser des Katheterrohres etwa 1 mm, während die Mittelöffnung einen Durchmesser von etwa 0,4 mm aufweist. Die Länge des Einsatzes beträgt etwa 25 mm. Die Ausnehmung weist an dem der Mittelöffnung zugewandten Ende den selben Durchmesser auf wie die Mittelöffnung. Der Außendurchmesser des Katheterkopfes beträgt am freien Ende etwa 10 mm. Die Dicke der Wand des Katheterkopfes beträgt etwa 1 mm. Der Intraperitonealkatheter weist eine Gesamtlänge von etwa 250 mm auf.

Vorzugsweise wird die Mittelöffnung 6 des zuvor erläuterten Katheters derart bemessen, daß sie geringfügig größer ist als der Außendurchmesser der in Längsrichtung durch den Einsatz geführten Einstechnadel 9. Auf diese Weise wird sichergestellt, daß die Einstechnadel 9 in die Mittelöffnung 6 eingeführt werden kann und diese dann etwa ausfüllt. Es ist auch denkbar, den Einsatz 5 in der Ausnehmung 4 derart zu befestigen bzw. zu verkleben, daß er nur einen Teil der Ausnehmung, nämlich den an die Mittelöffnung 6 angrenzenden Teil ausfüllt.

Im folgenden wird im Zusammenhang mit den Fig. 3 und 4 eine weitere Ausführungsform des vorliegenden Intraperitonealkatheters beschrieben, bei der der Katheterkopf 1' die Form eines rinnenförmigen Kastens aufweist, der in Längsrichtung durch Wände 1" abgeschlossen ist und dessen eine Wand 1" vorzugsweise einstückig derart an das Katheterrohr 7 angeformt ist, daß, wie dies insbesondere aus der Fig. 4 ersichtlich ist, der Boden der vorzugsweise etwa V-förmigen Rinne des Katheterkopfes 1 eine Verlängerung des unteren Bereiches des Rohres 7' darstellt. Nach oben öffnet sich diese Verlängerung entsprechend der tangential angreifenden Seitenwände des rinnenförmigen Katheterkopfes 1. Im Innern des rinnenförmigen Katheterkopfes 1 ist ein entsprechender V-förmiger Einsatz 5' angeordnet, dessen unteres Ende vorzugsweise so ausgebildet ist, daß es den oberen Bereich der Verlängerung des Katheterrohres 7' bildet. Der Einsatz 5' kann in dem rinnenförmigen Katheterkopf 1 verklebt sein oder es kann, wie dies bereits im Zusammenhang mit der ersten Ausführungsform beschrieben wurde, am oberen Ende des Katheterkopfes 1' ein nach innen ragender Flansch vorgesehen sein, der das obere Ende des Einsatzes 5' wenigstens teilweise übergreift.

Ein wesentlicher Vorteil dieser Ausführungsform besteht darin, daß der Katheterkopf 1' so an das Katheterrohr 7' angeformt ist, daß sich der Einstechbereich des Einsatzes 5' in der Längsrichtung des Rohres 7' erstreckt. Aufgrund der großen Fläche des Einstechbereiches des Einsatzes 5' können sehr viel Verbindungen zwischen der Einstechnadel und dem Katheter hergestellt werden.

Insbesondere ist es möglich, die axiale Länge des Katheterkopfes 1' und somit auch die Größe der Einstechfläche des Einsatzes 5' in Abhängigkeit von der geplanten Implantationszeit und der Anzahl der in dieser Zeit vorzunehmenden Einstechvorgänge zu bemessen.

Der Katheterkopf 1' und der Einsatz 5' der in den Fig. 3 und 4 dargestellten Ausführungsform können aus den Materialien bestehen, die im Zusammenhang mit der Ausführungsform der Fig. 1 und 2 angegeben wurden. Die Handhabung des Katheters der Fig. 3 und 4 ähnelt der Handhabung des Katheters der Fig. 1 und 2, wobei die Einstechnadel jedoch nicht in der Längsrichtung der Mittelöffnung 6 (Fig. 1, 2), sondern quer zur Längserstreckung der Verlängerung 6' zugeführt wird. Es ist auch denkbar, den Einsatz 5' nur in dem Bereich der Ausnehmung 4' anzuordnen, der unmittelbar an die Verlängerung des Katheterrohres 7' angrenzt.

Die vorliegende Erfindung ermöglicht erstmals aufgrund der besonderen Ausgestaltung des Katheterkofes eine direkte Verbindung zwischen dem Katheter und einer externen Arzneimittelabgabeeinrichtung, weil schädliche Arzneimittelvolumen entlang des Weges zwischen der Abgabeeinrichtung und dem Abgabeende des Katheters vermieden werden.

**Patentansprüche**

1. Intraperitonalkatheter für die Zuführung flüssiger Arzneimittel, insbesondere von Insulin, mit einem Katheterkopf (1, 1') und einem damit verbundenen Katheterrohr (7, 7') mit einer Mittelöffnung (6, 6'), wobei der Katheterkopf (1, 1') einen aus einem elastischen Material bestehenden in einer Ausnehmung (4, 4') angeordneten Einsatz (5, 5') aufweist, durch die von außen das flüssige Arzneimittel durch Hindurchführen einer Einstechnadel (9) durch den Einsatz (5, 5') einbringbar ist, dadurch gekennzeichnet, daß in der Ausnehmung (4,) unmittelbar angrenzend an die Mittelöffnung der die trichterförmige Ausnehmung (4,) ausfüllende Einsatz (5,) vorgesehen ist, und daß die trichterförmige Ausnehmung (4) koaxial an die Mittelöffnung angeformt ist, so daß die Einstechnadel (9) durch den Einsatz (5,) hindurch unmittelbar in die Mittelöffnung einführbar ist oder daß die Ausnehmung (4') durch einen Katheterkopf (1') gebildet ist, der die Form eines rinnenförmigen Kastens aufweist, dessen unterer, verengter Bereich eine Verlängerung des unteren Bereiches des Katheterrohres (7') bildet, wobei der Einsatz (5') derart geformt ist, daß zwischen seinem unteren Ende und dem unteren Bereich des Katheterkopfes (1') die Verlängerung der Mittelöffnung (6') des Katheterrohres (7') gebildet wird, so daß die Einstechnadel (9) durch den Einsatz (5') hindurch unmittelbar in den die Verlängerung der Mittelöffnung (6') des Katheterrohres (7') darstellenden Raum einführbar ist.

2. Katheter nach Anspruch 1, dadurch **gekennzeichnet**, daß die Ausnehmung (4) konisch ausgebildet ist, daß das an die Mittelöffnung (6) angrenzende Ende der Ausnehmung (4) etwa den selben Durchmesser aufweist wie die Mittelöffnung (6), daß die Ausnehmung (4) an der der Mittelöffnung (6) abgewandten Seite in einer Öffnung (3) endet, durch die die Einstechnadel (9) in den Einsatz (5) einführbar ist, der sich wenigstens in dem an die Mittelöffnung (6) angrenzenden Bereich der Ausnehmung (4) befindet.

3. Katheter nach Anspruch 2, dadurch **gekennzeichnet**, daß die Ausnehmung (4) trichterförmig ausgebildet ist.

4. Katheter nach Anspruch 2 oder 3, dadurch **gekennzeichnet**, daß der Einsatz (5) im wesentlichen die gesamte Ausnehmung (4) ausfüllt.

5. Katheter nach Anspruch 1, dadurch **gekennzeichnet**, daß der rinnenförmige Kasten (1') einen etwa V-förmigen Querschnitt aufweist.

6. Katheter nach einem der Ansprüche 2 bis 5, dadurch **gekennzeichnet**, daß der Einsatz (5, 5') die Ausnehmung (4, 4') des Katheterkopfes (1, 1') bis zu derem freien Ende ausfüllt, und daß am freien Ende der Ausnehmung (4, 4') ein radial nach innen ragender Flansch (11) angeformt ist, der den Randbereich des Einsatzes (5, 5') wenigstens teilweise übergreift.

7. Katheter nach einem der Ansprüche 1 bis 6, dadurch **gekennzeichnet**, daß der Einsatz (5, 5') in der Ausnehmung (4, 4') verklebt ist.

8. Katheter nach einem der Ansprüche 1 bis 7, dadurch **gekennzeichnet**, daß der Einsatz (5, 5') aus Silikongummi besteht.

9. Katheter nach einem der Ansprüche 1 bis 8, dadurch **gekennzeichnet**, daß der Katheterkopf (1) aus einem ein Einwachsen des Katheterkopfes (1) in das Hautgewebe ermöglichenden Material besteht.

**Claims**

1. Intraperitoneal catheter for administering liquid medication, particularly insulin, comprising a catheter head (1, 1') and a catheter tube (7, 7') connected therewith and having a central opening (6, 6'), said catheter head (1, 1') having an insert (5, 5') consisting of an elastic material and disposed in a recess (4, 4'), and said liquid medication being introduceable from the outside by passing a puncturing needle (9) through said insert (5, 5'), characterized in that insert (5) is provided in said recess (4) contiguous with said central opening, with insert (5) filling all of said funnel-shaped recess (4), and said funnel-shaped recess (4) being coaxially formed to said central opening so that puncturing needle (9) may be introduced through insert (5) directly into the central opening, or that recess (4') is formed by a catheter head (1') having the shape of a trough-like casing of which the lower narrowed portion forms an extension of the lower portion of catheter tube (7'), with insert (5') being shaped so that the extension of central opening (6') of catheter tube (7') is formed between its bottom end and the lower portion of catheter head (1'), whereby puncturing needle (9) may be introduced through insert (5') directly into the space forming the extension of central opening (6') of catheter tube (7').

2. Catheter as in claim 1, characterized in that recess (4) is conical in shape, in that the end of recess (4) adjacent central opening (6) has approximately the same diameter as central opening (6), in that recess (4) terminates at the side facing away from central opening (6) in an opening (3) through which puncturing needle (9) may be introduced in insert (5), said insert being located in at least the portion adjacent central opening (6) of recess (4).

3. Catheter as in claim 2, charaterized in that recess (4) is funnel-shaped.

4. Catheter as in claim 2 or 3, characterized in that insert (5) essentially fills all of recess (4).

5. Catheter as in claim 1, characterized in that said trough-shaped casing (1') has a substantially V-shaped cross section.

6. Catheter as in any one of claims 2 to 5, characterized in that insert (5, 5') fills recess (4, 4') of catheter head (1, 1') up to the free end thereof, and in that recess (4, 4') has formed at the free end thereof a radially inwardly projecting flange (11) at least partly overlying the marginal area of insert (5, 5').

7. Catheter as in any one of claims 1 to 6, characterized in that insert (5, 5') is bonded adhesively into recess (4, 4').

8. Catheter as in any one of claims 1 to 7, characterized in that insert (5, 5') consists of silicone rubber.

9. Catheter as in any one of claims 1 to 8, characterized in that catheter head (1) consists of a material enabling catheter head (1) to grow into the skin tissue.

**Revendications**

1. Cathéter intrapéritonéal pour l'administration de médicaments sous forme liquide, en particulier insuline, comprenant une tête de cathéter (1, 1') reliée à un tube de cathéter (7, 7') avec un passage central (6, 6'). La tête de cathéter (1, 1') présente un insert (5, 5') en matière élastique placé dans une cavité (4, 4') par laquelle on peut administrer le médicament liquide de l'extérieur par l'intermédiaire d'une aiguille d'introduction (9) qui passe par l'insert (5, 5'). Caractéristiques: l'insert (5) qui emplit la cavité en forme d'entonnoir (4) est conçu pour être au voisinage immédiat du passage central dans la cavité (4) et la cavité en forme d'entonnoir (4) occupe une position coaxiale par rapport au passage central, de telle sorte que l'aiguille d'introduction (9) peut être introduite directement dans le passage central en passant par l'insert (5); ou encore: la cavité (4') est formée par une tête de cathéter (1') en forme de gouttière rectangulaire dont la partie inférieure étroite constitue le prolongement de la partie inférieure du tube de cathéter (7'), l'insert (5') étant formé de telle sorte qu'entre son extrémité inférieure et la partie inférieure de la tête de cathéter (1'), on a le prolongement du passage central (6') du tube de cathéter (7') si bien que l'aiguille d'introduction (9) peut être introduite directement dans la zone constituant le prolongement du passage central (6') du tube de cathéter (7') en passant par l'insert (5').

2. Cathéter selon revendication 1, **caractérisé** par les faits suivants: la cavité (4) est de forme conique, l'extrémité de la cavité (4) attenante au passage central (6) a approximativement le même diamètre que le passage central (6), le côté de la cavité (4) opposé au passage central (6) débouche sur un orifice (3) qui permet d'introduire l'aiguille (9) dans l'insert (5), lequel se trouve au moins dans la zone de la cavité (4) attenante au passage central (6).

3. Cathéter selon revendication 2, **caractérisé** par le fait que la cavité (4) est de forme conique.

4. Cathéter selon revendication 2 ou 3, **caractérisé** par le fait que l'insert (5) occupe la quasi-totalité de l'espace de la cavité (4).

5. Cathéter selon revendication 1, **caractérisé** par le fait que la gouttière rectangulaire (1') est approximativement en forme de V en coupe transversale.

6. Cathéter selon l'une des revendications 2 à 5, **caractérisé** par les faits suivants: l'insert (5, 5') remplit entièrement la cavité (4, 4') de la tête de cathéter (1, 1') jusqu'à l'extrémité libre de cette cavité, et à l'extrémité libre de la cavité (4, 4') se trouve une bride dépassant radialement vers l'intérieur (11), laquelle recouvre au moins en partie le bord de l'insert (5, 5').

7. Cathéter selon l'une des revendications 1 à 6, **caractérisé** par le fait que l'insert (5, 5') est collé dans la cavité (4, 4').

8. Cathéter selon l'une des revendications 1 à 7, **caractérisé** par le fait que l'insert (5, 5') est en caoutchouc silicone.

9. Cathéter selon l'une des revendications 1 à 8, **caractérisé** par le fait que la tête de cathéter (1) est constituée d'une matière qui permet à la tête de cathéter (1) de s'incarner dans la peau.

H(Haut)

2

1

11

3

9

10

5

4

P(Bauchfell)

6

FIG. 1

7

B(Bauchhöhle)

2

1

11

3

6

4

5

FIG. 2

IV

5'

1"

1"

7'

6'

1'

IV

FIG. 3

5'

4'

1'

6'

FIG. 4

8